# EUROPÄISCHE PATENTSCHRIFT

(11) **EP 1 905 481 B1**
(45) Veröffentlichungstag und Bekanntmachung des Hinweises auf die Patenterteilung: **06.10.2010**
(21) Anmeldenummer: 07113946.3
(22) Anmeldetag: 07.08.2007
(51) Int. Cl.: A61N 5/10

(54) **Partikeltherapieanlage für die Partikeltherapie eines einer Bewegung ausgesetzten Zielvolumens**
Particle therapy facility for particle therapy of a target volume actuated by a movement
Installation de thérapie à particules pour la thérapie à particule d'un volume cible soumis à un mouvement

(30) Priorität: 29.09.2006 DE 102006046193; 29.09.2006 US 848602 P
(43) Veröffentlichungstag der Anmeldung: 02.04.2008
(73) Patentinhaber: SIEMENS AKTIENGESELLSCHAFT, 80333 München (DE)
(72) Erfinder: Rietzel, Eike, 64289, Darmstadt (DE)

(56) Entgegenhaltungen:
- EP-A- 0 826 394
- WO-A-02/41948
- WO-A-2006/094533
- DE-A1- 19 907 098
- US-B1- 6 265 837
- CHU W T ET AL: "INSTRUMENTATION FOR TREATMENT OF CANCER USING PROTON AND LIGHT-ION BEAMS" REVIEW OF SCIENTIFIC INSTRUMENTS, AIP, MELVILLE, NY, US, Bd. 64, Nr. 8, 1. August 1993 (1993-08-01), Seiten 2055-2122, XP000393868 ISSN: 0034-6748
- PHILLIPS M H ET AL: "Effects of respiratory motion on dose uniformity with a charged particle scanning method" PHYSICS IN MEDICINE AND BIOLOGY, TAYLOR AND FRANCIS LTD. LONDON, GB, Bd. 37, Nr. 1, 1. Januar 1992 (1992-01-01), Seiten 223-233, XP020021933 ISSN: 0031-9155
- MINOHARA S ET AL: "RESPIRATORY GATED IRRADIATION SYSTEM FOR HEAVY-ION RADIOTHERAPY" INTERNATIONAL JOURNAL OF RADIATION: ONCOLOGY BIOLOGY PHYSICS, PERGAMON PRESS, US, Bd. 47, Nr. 4, 1. Januar 2000 (2000-01-01), Seiten 1097-1103, XP001051093 ISSN: 0360-3016

## Beschreibung

Die Erfindung betrifft eine Partikeltherapieanlage zur Bestrahlung eines Patienten sowie ein Verfahren zur Bestrahlung eines Patienten.

Eine Partikeltherapieanlage weist üblicherweise eine Beschleunigereinheit und ein Hochenergiestrahlführungssystem auf. Die Beschleunigung der Partikel, z.B. Protonen, Pionen, Helium-, Kohlenstoff- oder Sauerstoff-Ionen, erfolgt beispielsweise mit Hilfe eines Synchrotrons, in das die üblicherweise mit einem Linearbeschleuniger vorbeschleunigten Partikel eingespeist werden, um dort auf die gewünschte Energie beschleunigt und für die Bestrahlung gespeichert zu werden. Alternativ kann die Beschleunigung auch über ein Zyklotron erfolgen, wobei verschiedene Energien z.B. über geeignete Energieabsorber ausgehend von einer Maximalenergie eingestellt werden können.

Ein Hochenergiestrahltransportsystem führt die Partikel von der Beschleunigereinheit zu einem oder mehreren Behandlungsräumen. Man unterscheidet zwischen "fixed beam"-Behandlungsräumen, in denen die Partikel aus einer festen Richtung auf den Behandlungsplatz treffen, und so genannten Gantry-basierten Behandlungsräumen. Bei letzteren ist es möglich, den Partikelstrahl aus verschiedenen Richtungen auf den Patienten zu richten.

Mit Hilfe einer Rasterscanvorrichtung wird der Partikelstrahl über einen Scanbereich verfahren. Dazu wird der Strahl, z.B. mit zwei Ablenkmagneten einstellbar, lateral versetzt. Die Bestrahlung erfolgt bevorzugt Volumenelement-orientiert, d.h., bei der Therapieplanung wird die zu applizierende Dosisverteilung aus auf ein Volumenelement orientierten Subdosen zusammengesetzt. Volumenelemente weisen üblicherweise Dimensionen von 2-3 mm auf (Strahldurchmesser und Iso-Energieschichtdicke), so dass der Form des zu bestrahlenden Bereichs, beispielsweise eines Tumors, gut gefolgt werden kann. Dabei werden die lateralen Ausmaße durch die Ionenstrahloptik eingestellt und das durch die Energieverteilung des Strahls bestimmte Ausmaß, d.h. die Tiefe des Volumenelements, durch einen entsprechenden Degrader, z.B. einen Ripple-Filter, eingestellt. Somit ergeben sich übliche Rasterabstände von 2-3 mm sowohl in lateraler Richtung als auch in Richtung der Eindringtiefe.

Ferner kann eine Therapieplanung eine Aufteilung des Bestrahlungsvorgangs in mehreren Einheiten vorsehen. Die Aufteilung kann zum einen geometrischer Natur sein, d.h. eine Aufteilung z.B. in aneinander angrenzende Bestrahlungsfelder, deren Größe durch das maximal scanbare Bestrahlungsfeld der Rasterscanvorrichtung gegeben ist. Zum anderen kann eine Aufteilung in zeitlich aufeinander folgende Bestrahlungseinheiten vorgenommen werden, um z.B. über mehrere Bestrahlungssitzungen verteilt eine entsprechende Wirkung zu erzielen.

Ein Kontroll- und Sicherheitssystem der Partikeltherapieanlage gewährleistet, dass jeweils ein mit den erbetenen Parametern charakterisierter Partikelstrahl in den entsprechenden Behandlungsraum geführt wird. Die Parameter werden im so genannten Behandlungsplan definiert, der angibt, wie viele Teilchen aus welcher Richtung mit welcher Energie auf den Patienten bzw. jedes der Volumenelemente treffen sollen. Die Energie der Partikel bestimmt die Eindringtiefe der Partikel in den Patienten, d.h. den Ort des Volumenelements, an dem das Maximum der Wechselwirkung mit dem Gewebe bei der Partikeltherapie erfolgt; in anderen Worten, den Ort, an dem das Maximum der Dosis deponiert wird. Die Energieverteilung des Partikelstrahls bestimmt somit die Dosisverteilung in Einfallsrichtung. Zusammen mit dem Strahlprofil bestimmt sie entsprechend die Ausmaße eines Bestrahlungsvolumens bei sonst unveränderten Strahlparametern.

Üblicherweise befinden sich vor dem Patienten Strahlüberwachungselemente, die beispielsweise die Lage und/oder die Intensität des Partikelstrahls überwachen. Die Lage des Partikelstrahls sowie sein Strahlprofil werden üblicherweise in Transmission mit Hilfe von geeigneten Detektoren, beispielsweise Ionisationskammern oder Vielkanalkammern, die sich im Strahlengang nahe am Patienten während der Behandlung befinden, gemessen.

Die Ausrichtung des Patienten zum Scanbereich der Partikeltherapieanlage erfolgt üblicherweise mithilfe einer Patientenpositioniervorrichtung im Behandlungsraum. Zur Positionsverifikation der Bestrahlungsposition eines vorzugsweise fixierten Patienten werden üblicherweise vor Bestrahlungsbeginn Durchleuchtungsbilder mit CT-Daten aus der Therapieplanung abgeglichen und der Patient evtl. nachjustiert. Eine Verschiebung des Patienten kann ferner den abscanbaren Bereich vergrößern.

Z.B. bei der Bestrahlung im Rasterscanverfahren wirkt sich die Bewegung eines Zielvolumens oder von durchstrahltem Volumen nachteilig auf die Homogenität der applizierten Partikelstrahlung aus, siehe z.B. M. H. Phillips et al., "Effects of respiratory motion on dose uniformity with a charged particle scanning method" Phys. Med Biol. 1992, Vol. 32, No 1, 223-234. Aufgrund von Interferenzen zwischen Strahlapplikation und Objektbewegung stimmen die applizierte Dosisverteilung und die geplante Dosisverteilung nicht mehr überein. Die applizierte Dosisverteilung weist aufgrund der Bewegung Inhomogenitäten auf, d.h., es kommt zu Unter- und/oder Überdosierungen.

Derartige Inhomogenitäten können durch mehrfaches Abscannen einer Energieschicht mit reduzierter Dosis verkleinert werden, da die Inhomogenitäten in ihrer Intensität kleiner ausfallen und an unterschiedlichen Stellen im Scanbereich vorliegen. Allerdings ist hierbei eine Sicherheitszone um das zu bestrahlende Gewebe zu legen, welcher die Amplituden der Bewegung berücksichtigt und somit sicherstellt, dass das zu bestrahlende Gewebe ausreichend bestrahlt wird. Ferner stellt sich das Problem, dass aneinander grenzende Energieschichten unterschiedlich von der Bewegung betroffen sind, so dass sich zusätzlich Inhomogenitäten in der Z-Richtung, d.h. in Richtung des einfallenden Strahls, ergeben können.

Die Bestrahlung von sich z.B. aufgrund der Atmung bewegenden Objekte durch bewegungsabhängiges Ausblenden (Gating) des Therapiestrahls umgeht die Auswirkung der Bewegung, durch die Einschränkung der Bestrahlung auf quasi-stationäre Bewegungszustände, so dass die laterale Sicherheitszone reduziert werden kann. Eine derartige Partikeltherapieanlage ist z.B. aus S. Minohara et al., "Respiratory Gated Irradiation System for Heavy-ion Radiotherapy", Int. J. Radiation Oncology Biol. Phys., Vol. 47, No. 4, pp. 1097-1103, 2000 bekannt. Ähnlich bewirkt ein Anhalten der Atmung quasistationäre Zustände, wobei allerdings -wie auch beim Gating- noch immer Inhomogenitäten in der Dosisverteilung, insbesondere im Übergangsbereich aneinander grenzender Energieschichten, entstehen können.

Eine Aufgabe der Erfindung ist es, die Bestrahlung von bewegten Objekten bei der Partikeltherapie mittels Rasterscanverfahren zu verbessern.

Die Aufgabe wird durch eine Partikeltherapieanlage nach Anspruch 1 und durch ein Scanverfahren nach Anspruch 12 gelöst.

Eine Partikeltherapieanlage nach der Erfindung weist einen Partikelbeschleuniger zur Bereitstellung von hochenergetischen Partikeln, eine Einstellvorrichtung zum Einstellen einer Größe des Bestrahlungsvolumens, ein Kontrollsystem zur Ansteuerung der Einstellvorrichtung sowie eine Scanvorrichtung auf. Die Einstellvorrichtung, z.B. stellvertretend für die Optik in der Strahlführung der Partikel sowie für eine Anpassvorrichtung zum Anpassen einer Energieverteilung der Partikel wird dabei vom Kontrollsystem derart angesteuert, dass eine Größe eines Bestrahlungsvolumens eingestellt wird, die es ermöglicht, während eines Zeitintervalls, in dem beispielsweise eine Atembewegung angehalten (oder verlangsamt) wird, das gesamte Zielvolumen abzuscannen. Bei diesem Scanvorgang wird mindestens eine Energieschicht mit dem Bestrahlungsvolumen gemäß dem Verlauf des Zielvolumens bestrahlt.

Dieser Ausbildung einer Partikeltherapieanlage liegt die Erkenntnis zugrunde, dass die üblichen Bestrahlungsvolumina bei Scanverfahren zwar eine gute Ortsauflösung ermöglichen, aber entsprechend lange Bestrahlungszeiten erfordern, in denen sich Bewegungen negativ auf die Homogenität der Dosisverteilung auswirken können. Entsprechend wird erfindungsgemäß das Bestrahlungsvolumen so angepasst, dass auch in einem kurzen Zeitraum das gesamte Zielvolumen gescannt werden kann, so dass sich bei der Bestrahlung keine Grenzverläufe zwischen aufeinander folgenden Bestrahlungsvorgängen ergeben. Insbesondere ist es vorteilhaft, wenn sog. Nadelstrahlen (pencil beams) mit geringem Querschnitt durch einen geeigneten Absorber in der Tiefe aufgeweitet werden. D.h. die Energieverteilung wird beispielsweise über einen sog. Ripplefilter verbreitert, so dass sich eine in Einfallsrichtung des Strahls erweitere Brag-Peak-Zone ergibt. Entsprechend sind nur wenige Energieschichten zu bestrahlen, was innerhalb des Zeitintervalls möglich ist. Die Ortsauflösung in lateraler Richtung bleibt im Wesentlichen unverändert, und der Strahl wird in lateraler Richtung schnell gescannt. Die Scangeschwindigkeit wird dabei so gewählt, dass für den zu behandelnden Patienten das gesamte Zielvolumen während einer einzigen Phase der beispielsweise arretierten Atmung bestrahlt wird. Dieser Vorgang wird jeweils - bei angehaltener Atmung - so oft wiederholt, bis die erforderliche Dosis im Zielgebiet deponiert ist. Durch beispielsweise eine passive Energievariation während eines Bestrahlungsvorgangs in einem Zeitintervall können mehrere erforderliche Energieschichten bestrahlt werden. Alternativ oder ergänzend zu passiven Energievariationen kann auch die Beschleunigung im Synchrotron variiert werden.

Eine derartige Partikeltherapieanlage erlaubt es, dass, obwohl in den quasistationären Zeitintervallen - z.B. beim Gating oder beim Anhalten der Atmung - Bewegungszustände bestrahlt werden, die nicht exakt gleich sind, trotzdem keine Unter - bzw. Überdosierungen entstehen, da in aufeinander folgenden Zeitintervallen weder Energieschichten noch laterale Bereiche aneinander gesetzt werden, um die gewünschte Dosisverteilung zu erreichen. Entsprechend umgeht eine derartige Partikeltherapieanlage den Nachteil, dass einzelne Bestrahlungspunkte, Bestrahlungsbereiche und auch Energieschichten nicht wie gewünscht zusammenpassen. Denn erfolgt ein Abscanvorgang jeweils über das komplette Volumen im selben Patientenzustand, können sich Abweichungen von der geplanten Dosis nur am Rand des Bestrahlungsfeldes ergeben. Innerhalb des Zielvolumens treten keine signifikanten Dosisschwankungen auf. So ermöglicht eine zusätzliche Vergrößerung, d.h. Aufweitung und/oder Vertiefung, des Bestrahlungsvolumens eine schnelle Applikation eines kompletten Feldes aufgrund der reduzierten Anzahl von Rasterpunkten und/oder Iso-Energieschichten.

Eine besonders günstige Einstellung des Bestrahlungsvolumens liegt vor, wenn das Verhältnis von distalem zu lateralem Ausmaß des Bestrahlungsvolumens größer gleich 2 zu 1 ist.

Ferner ist es besonders vorteilhaft, das Bestrahlungsvolumen auf ein Ausmaß in Richtung des Strahleinfalls einzustellen, welches mindestens 10 % des distalen Ausmaßes des Zielvolumens aufweist. Dies erlaubt es, das Zielvolumen mit 10 bzw. bevorzugt 5 bis 3 Isoenergieschichten zu scannen. Beispielsweise liegt das das distale Ausmaß in einem Bereich zwischen 5 und 25 mm.

Als Scanvorrichtung kann beispielsweise eine Rasterscanvorrichtung für einen Pencil-Beam verwendet werden.

Ferner wird die Erfindung durch ein Scanverfahren gelöst, bei dem ein einer (Atem-)Bewegung ausgesetztes Zielvolumen eines Patienten mit einem Partikelstrahl bestrahlt werden soll, wobei ein Bestrahlungsvolumen, in dem Partikel mit dem Gewebe zur Therapie wechselwirken, über das Zielvolumen gescannt wird. In dem Verfahren wird die Bewegung des Zielvolumens angehalten (oder verlangsamt), um in einem Zeitintervall ein quasi-stationäres Zielvolumen zu schaffen. In diesem Zeitintervall ist mit bisherigen Bestrahlungsvolumina nur ein Teilbereich des Zielvolumens bestrahlbar. Entsprechend wird erfindungsgemäß das Bestrahlungsvolumen auf ein Ausmaß eingestellt, das es erlaubt, während des Zeitintervalls das gesamte zu bestrahlende Volumen zu bestrahlen. In einem solchen Zeitintervall wird dann das gesamte zu bestrahlende Zielvolumen mit dem entsprechend eingestellten Bestrahlungsvolumen abgescannt, wobei der Vorgang sooft wiederholt wird, bis die erforderliche Dosis appliziert wurde. Das Verfahren hat ferner den Vorteil, dass Inhomogenitäten in der applizierten Dosisverteilung vermieden werden.

Weitere vorteilhafte Ausführungsformen der Partikeltherapieanlage und des Verfahrens sind durch die Merkmale der Unteransprüche gekennzeichnet. Es folgt die Erläuterung von mehreren Ausführungsbeispielen der Erfindung anhand der Figuren 1 bis 6. Es zeigen:
- FIG 1: eine schematische Partikeltherapieanlage,
- FIG 2: eine Anordnung von Bestrahlungsvolumina nach dem Stand der Technik,
- FIG 3: eine Dosisverteilung in Einfallsrichtung,
- FIG 4: eine Aufteilung eines Zielvolumens in Bestrahlungsvolumina zur Bestrahlung innerhalb eines Zeitintervalls,
- FIG 5: eine zu Figur 4 gehörende Dosisverteilung in Einfallsrichtung und
- FIG 6: eine Aufteilung eines Zielvolumens in Bestrahlungsvolumina und unter Verwendung von Aufweitungselementen.

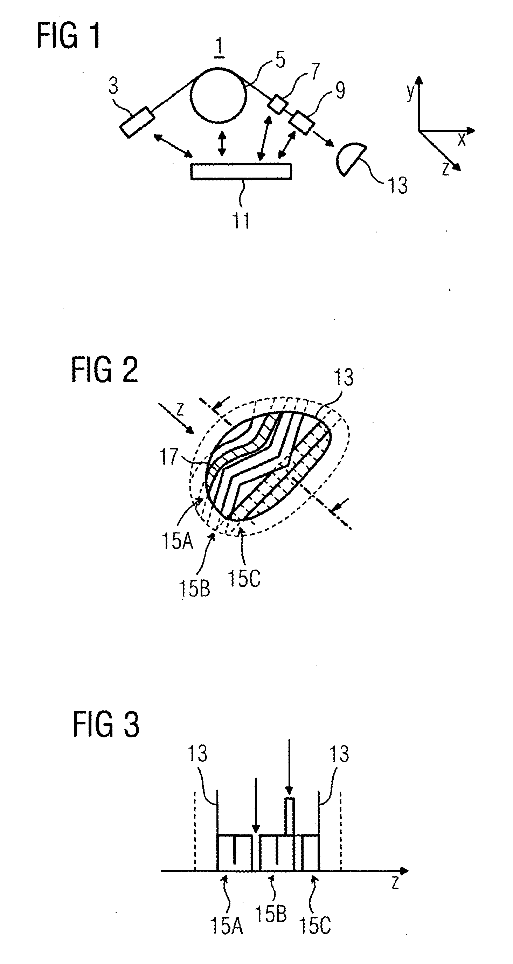

Figur 1 zeigt eine Partikeltherapieanlage 1 mit einem Vorbeschleuniger 3 und einem Synchrotron 5 sowie einer Scanvorrichtung 7 und einer Einstellvorrichtung 9. Ein Kontrollsystem 11 stellt die Energie der Partikel ein, die vom Synchrotron 5 ausgekoppelt werden. Die Scanvorrichtung 7 ermöglicht laterale Ablenkungen des Partikelstrahls in X- und Y-Richtung; sie wird beispielsweise ebenfalls vom Kontrollsystem 11 angesteuert. Die Einstellvorrichtung 9 ist beispielsweise ein Satz von Aufweitungselementen, beispielsweise Ripple-Filtern, die den Strahl in seiner Energieverteilung beispielsweise um einen Faktor 1, 2, 4 und 8 verbreitern. Entsprechend sind Bestrahlungsvolumina einstellbar, die in Z-Richtung, d.h. in Richtung des Strahls (distal), um einen Faktor 2, 4 oder 8 gestreckt sind.

Üblicherweise werden in der Partikeltherapie Bestrahlungsvolumina mit Ausmaßen in X-, Y- und Z-Richtung von (3 mm, 3 mm, 2 mm) oder (5 mm, 5 mm, 3-5 mm) verwendet. Diese erlauben ein konturiertes Anpassen des bestrahlten Volumens an das zu bestrahlende Zielvolumen 13. Befindet sich das Zielvolumen nun z.B. in der Nähe der Lunge und wird somit beim Atmen bewegt, ändert sich die Lage des Zielvolumens 13 und des durchstrahlten Gewebes im Einfallskanal des Partikelstrahls. Entsprechend ändert sich der Verlauf einer Iso-Energieschicht während eines Atmungszyklus.

Dies zeigt Figur 2 schematisch anhand von drei verformten Paaren von Iso-Energieschichten 15A, 15B und 15C bei unterschiedlichen Bewegungszuständen. Werden diese Schichten beispielsweise in aufeinander folgenden Zeitintervallen eingestrahlt, ergeben sich Inhomogenitäten in der Dosisverteilung aufgrund der Überlappung oder der fehlenden Bündigkeit der verformten Iso-Energieschichten im Zielvolumen 13. Schematisch wurde in Figur 2 in die Iso-Energieschicht 15A ein symmetrisches Bestrahlungsvolumen 17 eingezeichnet, das beispielsweise Ausmaße von 3 mm in lateraler und Z-Richtung aufweist. Das Ausmaß des Zielvolumens 13 in Z-Richtung ist beispielsweise acht Iso-Energieschichtdicken, wobei um Bewegungsverschiebungen auszugleichen eine Sicherheitszone mit einer Gesamtdicke von einer Iso-Energieschicht um das Zielvolumen 13 mitbestrahlt wird.

Figur 3 zeigt zur Verdeutlichung zur Figur 2 wie beispielsweise zwischen der Iso-Energieschicht 15A und 15B eine nicht bestrahlte Zone verbleibt und wie sich die Energieschicht 15B und die Energieschicht 15C zur doppelten applizierten Dosis überlagern. Derartige Inhomogenitäten in Z-Richtung sind auch durch wiederholtes (konventionelles) Rescanning nicht zu vermeiden.

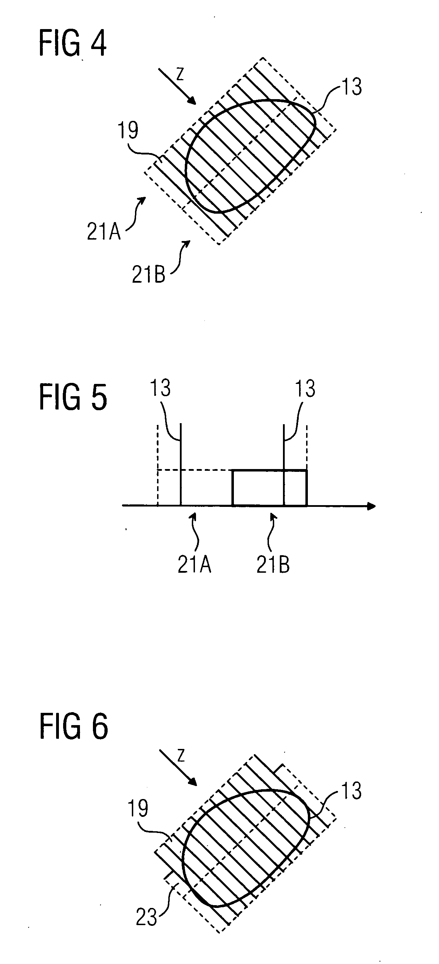

Figur 4 zeigt nun eine Aufteilung von Bestrahlungsvolumina 19 auf das Zielvolumen 13 mit Hilfe eines Aufweitungselements, das ein Bestrahlungsvolumen 19 mit einer viermal größeren Länge in Z-Richtung als in lateraler Richtung aufweist und so beispielsweise in Z-Richtung eine Länge von 12 mm und eine laterale Ausdehnung von 3 mm aufweisen. Bei dieser Größe des Bestrahlungsvolumens 19 ist es möglich, das gesamte Zielvolumen 13 in einem Zeitintervall, in dem die (Atem-) Bewegung angehalten wird, mit zwei Iso-Ergieschichten 21A und 21B zu bestrahlen. Entsprechend gibt es keine Dosisvariationen bei der Bestrahlung, da das gesamte Zielvolumen einmal unter identischen Bedingungen bestrahlt wird.

Figur 5 zeigt wie aufgrund der Bestrahlung des gesamten Zielvolumens in einem Zeitintervall keine bewegungsbedingten Inhomogenitäten entstehen. Man erkennt die Grenzen des Zielvolumens 13, dessen Inneres mit einer umgebenden Sicherheitszone mittels der beiden Iso-Energieschicht 21A und 21B homogen bestrahlt wird.

Ein realsierbares Zeitintervall z.B. für eine Atemunterbrechung liegt bevorzugt in der Größenordnung von 20 s, so dass auch größere Zielvolumina als Ganzes abgescannt werden können, wenn sich die Anzahl der Iso-Energieschichten z.B. um einen Faktor 4 reduziert.

Figur 6 verdeutlicht die Verwendung von mehreren Aufweitungselementen 19, wodurch ortsabhängig z.B. im Randbereich das Ausmaß des Bestrahlungsvolumens in Z-Richtung reduziert werden kann, um die Sicherheitszone um das Zielgewebe 13 nicht unnötig groß auszubilden. Dazu werden im Randbereich anstelle der um den Faktor vier gestreckten Bestrahlungsvolumina 19 nur um den Faktor zwei gestreckte Bestrahlungsvolumina 23 verwendet. Dazu werden ortsabhängig verschiedene Aufweitungselemente in den Strahl eingebracht bzw. die Beschleunigung/Energieverteilung entsprechend angepasst.

Wird nun mit diesem Vorgehen das Zielvolumen 13 mehrfach als Ganzes in Einem bestrahlt, ergibt sich auch für die Partikeltherapie bewegter Ziele im Scanverfahren eine homogene Dosisverteilung über das Zielvolumen 13.

## Patentansprüche

1. Partikeltherapieanlage (1) für die Partikeltherapie eines einer Bewegung ausgesetzten Zielvolumens (13) eines Patienten,
- mit einem Partikelbeschleuniger zur Bereitstellung von hochenergetischen Partikeln,
- mit einer Einstellvorrichtung (9) zur Einstellung einer Größe eines Bestrahlungsvolumens, in dem Partikel mit dem Gewebe zur Therapie wechselwirken
- mit einem Scanvorrichtung (7), und
- mit einem Kontrollsystem (11) zur Ansteuerung der Einstellvorrichtung, **dadurch gekennzeichnet, dass** das Kontrollsystem (11) ausgebildet ist zur Ausgabe eines Steuersignals an die Einstellvorrichtung, welches die dreidimensionalen Ausmaße des Bestrahlungsvolumens (17) derart einstellt, dass während eines Zeitintervalls von 20 s das gesamte Zielvolumen bestrahlt wird, wobei die Scanvorrichtung (7) derart angesteuert wird dass das gesamte Zielvolumen mit dem Bestrahlungsvolume (17) im Zeitintervall abgescannt wird.

2. Partikeltherapieanlage (1) nach Anspruch 1, wobei die Einstellvorrichtung (9) eine Anpassvorrichtung zum Anpassen einer Energieverteilung des applizierten Partikelstrahls, welche das distale Ausmaß des Bestrahlungsvolumens (17) bestimmt, aufweist.

3. Partikeltherapieanlageieanlage (1) nach Anspruch 1 oder 2, wobei die Einstellvorrichtung (9) eine Hochenergiestrahlführung zur Anpassung der lateralen Ausmaße des applizierten Partikelstrahls mittels Strahlführungsoptik aufweist.

4. Partikeltherapieanlage (1) nach einem der vorhergehenden Ansprüche, wobei die Einstellvorrichtung (9) ausgebildet ist zur Einstellung des Bestrahlungsvolumens (17) auf ein distal-zulateral-Verhältnis der Ausmaße von größer gleich 2 zu 1.

5. Partikeltherapieanlage (1) nach einem der vorhergehenden Ansprüche, wobei die Einstellvorrichtung (9) ausgebildet ist zur Einstellung des Bestrahlungsvolumens (17) auf ein Ausmaß in Richtung des Strahleinfalls, welches mindestens 10% des distalen Ausmaßes des Zielvolumens aufweist.

6. Partikeltherapieanlage (1) nach einem der vorhergehenden Ansprüche, wobei die Einstellvorrichtung (9) ausgebildet ist zur Einstellung des Bestrahlungsvolumens (17) auf ein Ausmaß in Richtung des Strahleinfalls im Bereich zwischen 3 mm und 25 mm.

7. Partikeltherapieanlage (1) nach einem der vorhergehenden Ansprüche, wobei die Einstellvorrichtung (9) ausgebildet ist zur Einstellung des Bestrahlungsvolumens (17) auf ein laterales Ausmaß im Bereich zwischen 5 mm und 10 mm.

8. Partikeltherapieanlage (1) nach einem der vorhergehenden Ansprüche, wobei die Anpassvorrichtung ein Degrader, insbesondere ein Ripple-Filter ist.

9. Partikeltherapieanlage (1) nach einem der vorhergehenden Ansprüche, wobei die Scanvorrichtung (7) eine Rasterscanvorrichtung ist.

10. Partikeltherapieanlage (1) nach einem der vorhergehenden Ansprüche, wobei der Partikelbeschleuniger zum Einstellen der Energie für die Bestrahlung mehrerer Energieschichten in einem Zeitintervall ausgebildet ist.

11. Partikeltherapieanlage (1) nach einem der vorhergehenden Ansprüche, wobei ferner Mittel zum Einstellen der Energie für die Bestrahlung mehrerer Energieschichten vorgesehen sind.

## Claims

1. Particle therapy facility (1) for particle therapy of a target volume (13) of a patient which is exposed to a movement,
- having a particle accelerator for the provision of high-energy particles,
- having an adjusting device (9) for adjusting a variable of an irradiation volume, in which particles interact with the tissue for therapy,
- having a scanning device (7), and
- having a control system (11) for triggering the adjusting device, **characterised in that** the control system (11) is designed to output a control signal to the adjusting device, said signal adjusting the three-dimensional sizes of the irradiation volume (17) so that the entire target volume is irradiated during a time interval of 20 s,
wherein the scanning device (7) is triggered so that the entire target volume is scanned with the irradiation volume (17) in the time interval.

2. Particle therapy facility (1) according to claim 1, wherein the adjusting device (9) has an adaptation device to adapt an energy distribution of the applied particle beam, which determines the distal size of the irradiation volume (17).

3. Particle therapy facility (1) according to claim 1 or 2, wherein the adjusting device (9) has a high-energy beam guide to adapt the lateral sizes of the applied particle beam by means of an optical beam guide.

4. Particle therapy facility (1) according to one of the preceding claims, wherein the adjusting device (9) is designed to adjust the irradiation volume (17) to a distal-to-lateral ratio of the sizes of greater than or equal to 2 to 1.

5. Particle therapy facility (1) according to one of the preceding claims, wherein the adjusting device (9) is designed to adjust the irradiation volume (17) to a size in the direction of the beam incidence, said size having at least 10% of the distal size of the target volume.

6. Particle therapy facility (1) according to one of the preceding claims, wherein the adjusting device (9) is designed to adjust the irradiation volume (17) to a size in the direction of the beam incidence in the range between 3 mm and 25 mm.

7. Particle therapy facility (1) according to one of the preceding claims, wherein the adjusting device (9) is designed to adjust the irradiation volume (17) to a lateral size in the range between 5 mm and 10 mm.

8. Particle therapy facility (1) according to one of the preceding claims, wherein the adaptation device is a degrader, in particular a ripple filter.

9. Particle therapy facility (1) according to one of the preceding claims, wherein the scanning device (7) is a raster scanning device.

10. Particle therapy facility (1) according to one of the preceding claims, wherein the particle accelerator is designed to adjust the energy for the irradiation of a plurality of energy layers in a time interval.

11. Particle therapy facility (1) according to one of the preceding claims, wherein furthermore means are provided for adjusting the energy for the irradiation of a plurality of energy layers.

## Revendications

1. Installation de thérapie particulaire (1) pour la thérapie particulaire d'un volume cible (13) d'un patient, exposé à un mouvement, comprenant
- un accélérateur de particules pour fournir des particules riches en énergie ;
- un dispositif de réglage (9) pour le réglage d'une dimension d'un volume d'irradiation, dans lequel des particules entrent en interaction avec le tissu à des fins de thérapie ;
- un dispositif à balayage (7) ; et
- un système de contrôle (11) pour l'entraînement du dispositif de réglage, **caractérisé en ce que** le système de contrôle (11) est réalisé pour envoyer un signal de commande au dispositif de réglage, qui règle la mesure en trois dimensions du volume d'irradiation (17) de telle sorte qu'au cours d'un intervalle de temps de 20 secondes, le volume cible total est irradié, le dispositif à balayage (7) étant entraîné de telle sorte que le volume cible total est balayé avec le volume d'irradiation (17) dans l'intervalle de temps.

2. Installation de thérapie particulaire (1) selon la revendication 1, dans laquelle le dispositif de réglage (9) présente un dispositif d'adaptation pour l'adaptation d'une distribution d'énergie du rayon particulaire appliqué qui détermine l'étendue distale du volume d'irradiation (17).

3. Installation de thérapie particulaire (1) selon la revendication 1 ou 2, dans laquelle le dispositif de réglage (9) présente un guidage du rayon riche en énergie à des fins d'adaptation de l'étendue latérale du rayon particulaire appliqué au moyen d'une optique de guidage du rayon.

4. Installation de thérapie particulaire (1) selon l'une quelconque des revendications précédentes, dans laquelle le dispositif de réglage (9) est réalisé pour le réglage du volume d'irradiation (17) en se basant sur un rapport distal/latéral dont la dimension est supérieure ou égale à 2/1.

5. Installation de thérapie particulaire (1) selon l'une quelconque des revendications précédentes, dans laquelle le dispositif de réglage (9) est réalisé pour le réglage du volume d'irradiation (17) en se basant sur une mesure dans la direction de l'incidence du rayon, qui présente au moins 10 % en de l'étendue distale du volume cible.

6. Installation de thérapie particulaire (1) selon l'une quelconque des revendications précédentes, dans laquelle le dispositif de réglage (9) est réalisé pour le réglage du volume d'irradiation (17) en se basant sur une mesure dans la direction de l'incidence du rayon dans la plage entre 3 mm et 25 mm.

7. Installation de thérapie particulaire (1) selon l'une quelconque des revendications précédentes, dans laquelle le dispositif de réglage (9) est réalisé pour le réglage du volume d'irradiation (17) en se basant sur une étendue latérale dans la plage entre 5 mm et 10 mm.

8. Installation de thérapie particulaire (1) selon l'une quelconque des revendications précédentes, dans laquelle le dispositif d'adaptation est un agent de dégradation, en particulier un filtre d'ondulation.

9. Installation de thérapie particulaire (1) selon l'une quelconque des revendications précédentes, dans laquelle le dispositif à balayage (7) est un dispositif à balayage de trame.

10. Installation de thérapie particulaire (1) selon l'une quelconque des revendications précédentes, dans laquelle l'accélérateur de particules est réalisé pour régler l'énergie pour l'irradiation de plusieurs couches d'énergie dans un intervalle de temps.

11. Installation de thérapie particulaire (1) selon l'une quelconque des revendications précédentes, dans laquelle on prévoit des moyens supplémentaires pour le réglage de l'énergie pour l'irradiation de plusieurs couches d'énergie.
